# EUROPÄISCHE PATENTSCHRIFT

(11) **EP 3 971 195 B1**
(45) Veröffentlichungstag und Bekanntmachung des Hinweises auf die Patenterteilung: **22.02.2023**
(21) Anmeldenummer: 20197484.7
(22) Anmeldetag: 22.09.2020
(51) Int. Cl.: C07F 9/6574, C07C 45/50, C07C 47/02

(54) **DIPHOSPHITE MIT EINEM OFFENEN, 2,4-METHYLIERTEN FLÜGELBAUSTEIN**
DIPHOSPHITES HAVING AN OPEN 2,4-METHYLATED BUILDING BLOCK
DIPHOSPHITE COMPRENANT UN COMPOSANT PÉRIPHÉRIQUE 2,4-MÉTHYLÉ OUVERT

(43) Veröffentlichungstag der Anmeldung: 23.03.2022
(73) Patentinhaber: Evonik Operations GmbH, 45128 Essen (DE)
(72) Erfinder: SALE, Anna Chiara, 45657 Recklinghausen (DE); FRANKE, Robert, 45772 Marl (DE); BRÄCHER, Alexander, 45721 Haltern am See (DE); FRIDAG, Dirk, 45721 Haltern am See (DE); KNOSSALLA, Johannes, 46514 Schermbeck (DE)
(74) Vertreter: Evonik Patent Association

(56) Entgegenhaltungen:
- EP-A2- 0 213 639
- JP-A- 2014 189 525

## Beschreibung

Die Erfindung betrifft Diphosphite mit einem offenen, 2,4-methylierten Flügelbaustein und deren Verwendung in der Hydroformylierung.

Phosphorhaltige Verbindungen spielen als Liganden in einer Vielzahl von Reaktionen eine entscheidende Rolle, z.B. in der Hydrierung, in der Hydrocyanierung und auch in der Hydroformylierung.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigem Phosphor P^{III}. Eine gute Übersicht über den Stand der Hydroformylierung von Olefinen findet sich in R. Franke, D. Selent, A. Börner, "Applied Hydroformylation", Chem. Rev., 2012, DOI:10.1021/cr3001803.

In EP 0213639 A2 ist auf Seite 98 in Beispiel 10 die folgende Verbindung dargestellt:

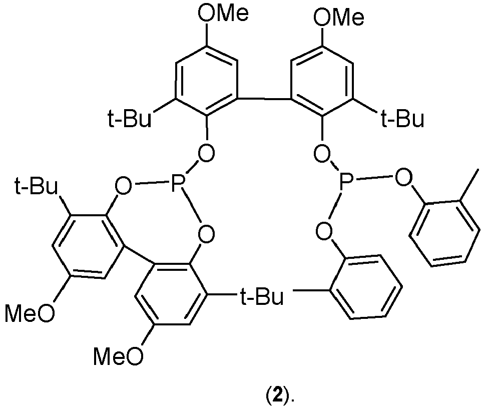

Die Verbindung (2) wird hier als Ligand in der Hydroformylierung von 1-Buten eingesetzt.

Die technische Aufgabe der Erfindung ist die Bereitstellung neuer Liganden, welche in der Hydroformylierung von Olefinen eine gesteigerte n/iso-Selektivität verglichen zu dem aus dem Stand der Technik bekennten Liganden aufweisen.

Die Aufgabe wird gelöst durch eine Verbindung gemäß Anspruch 1.

Verbindung gemäß der Struktur (**I**): 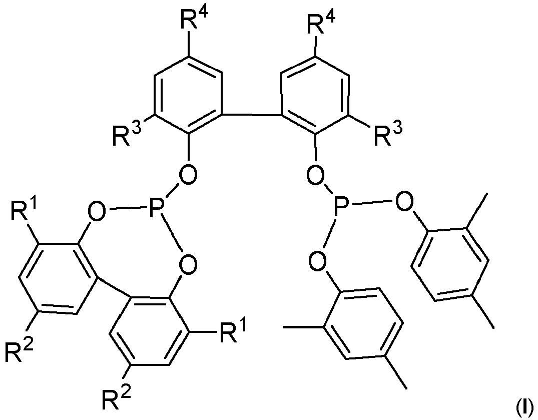 wobei R¹, R², R³, R⁴ ausgewählt sind aus: -H, -(C₁-C₁₂)-Alkyl, -O-(C₁-C₁₂)-Alkyl.

In einer Ausführungsform sind R¹, R³ ausgewählt aus: -H, -(C₁-C₁₂)-Alkyl.

In einer Ausführungsform stehen R¹, R³ für-(C₁-C₁₂)-Alkyl.

In einer Ausführungsform stehen R¹, R³ für -tBu.

In einer Ausführungsform stehen R¹, R³ für den gleichen Rest.

In einer Ausführungsform sind R², R⁴ ausgewählt aus: -H, -O-(C₁-C₁₂)-Alkyl.

In einer Ausführungsform stehen R², R⁴ für -O-(C₁-C₁₂)-Alkyl.

In einer Ausführungsform stehen R², R⁴ für -OMe.

In einer Ausführungsform stehen R², R⁴ für den gleichen Rest.

In einer Ausführungsform stehen R¹, R², R³, R⁴ nicht alle gleichzeitig für -CH₃.

In einer Ausführungsform weist die Verbindung die Struktur (1) auf:

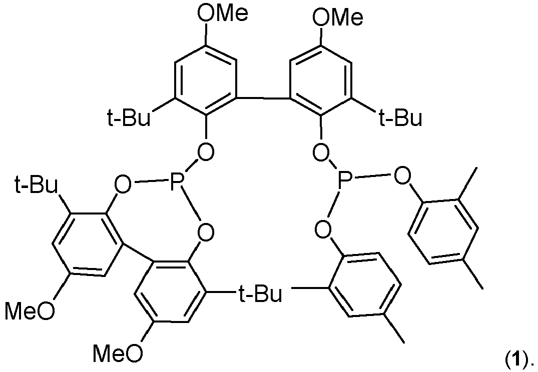

Neben der Verbindung als solche wird auch deren Verwendung zur Katalyse einer Hydroformylierungsreaktion beansprucht.

Verwendung einer zuvor beschriebenen Verbindung in einem Ligand-Metall-Komplex zur Katalyse einer Hydroformylierungsreaktion.

Des Weiteren wird ein Verfahren beansprucht, in welchem die zuvor beschriebene Verbindung als Ligand eingesetzt wird.

Verfahren umfassend die Verfahrensschritte:
a) Vorlegen eines Olefins,
b) Zugabe einer zuvor beschriebenen Verbindung und einer Substanz, welche ein Metall ausgewählt aus: Rh, Ru, Co, Ir aufweist,
c) Zuführen von H₂ und CO,
d) Erwärmen des Reaktionsgemisches aus a) bis c), wobei das Olefin zu einem Aldehyd umgesetzt wird.

In einer bevorzugten Ausführungsform ist das Metall Rh.

Es kann hierbei auch ein Überschuss an Liganden verwendet werden und nicht zwangsläufig jeder Ligand liegt gebunden in Form eines Ligand-Metall-Komplexes vor, sondern ist als freier Ligand im Reaktionsgemisch enthalten.

Die Reaktion wird bei üblichen Bedingungen durchgeführt.

Bevorzugt sind eine Temperatur von 80 °C bis 160 °C und ein Druck von 10 bis 60 bar. Besonders bevorzugt sind eine Temperatur von 100 °C bis 140 °C und ein Druck von 20 bis 50 bar.

Die Edukte für die Hydroformylierung gemäß dem Verfahren der Erfindung sind Olefine oder Gemische von Olefinen, insbesondere Monoolefine mit 2 bis 24, bevorzugt 3 bis 16, besonders bevorzugt 3 bis 12 Kohlenstoffatomen mit end- oder innenständigen C-C-Doppelbindungen, wie z.B. 1-Propen, 1-Buten, 2-Buten, 1- oder 2-Penten, 2-Methyl-1-buten, 2-Methyl-2-buten, 3-Methyl-1-buten, 1-, 2- oder 3,-Hexen, das bei der Dimerisierung von Propen anfallende Cs-Olefingemisch (Dipropen), Heptene, 2- oder 3-Methyl-1-hexene, Octene, 2-Methylheptene, 3-Methylheptene, 5-Methyl-2-hepten, 6-Methyl-2-hepten, 2-Ethyl-1-hexen, das bei der Dimerisierung von Butenen anfallende Cs-Olefingemisch (Di-n-buten, Di-iso-buten), Nonene, 2- oder 3-Methyloctene, das bei der Trimerisierung von Propen anfallende C₉-Olefingemisch (Tripropen), Decene, 2-Ethyl-1-octen, Dodecene, das bei der Tetramerisierung von Propen oderderTrimerisierung von Butenen anfallende C₁₂-Olefingemisch (Tetrapropen oder Tributen), Tetradecene, Hexadecene, das bei der Tetramerisierung von Butenen anfallende C₁₆-Olefingemisch (Tetrabuten) sowie durch Cooligomerisierung von Olefinen mit unterschiedlicher Anzahl von Kohlenstoffatomen (bevorzugt 2 bis 4) hergestellte Olefingemische.

Mit dem erfindungsgemäßen Verfahren können unter Verwendung der erfindungsgemäßen Liganden α-Olefine, endständig verzweigte, innenständige und innenständig verzweigte Olefine hydroformyliert werden.

Im Folgenden soll die Erfindung anhand von Ausführungsbeispielen näher erläutert werden.

### Arbeitsvorschriften

### Allgemeine Analytik

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet.

Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR³¹P = SR¹H * (BF³¹P / BF¹H) = SR¹H * 0,4048.

### Synthese (1)

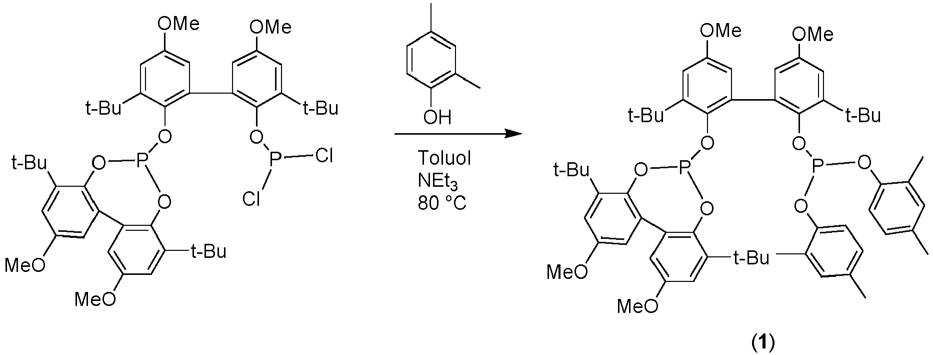

In der Glove-Box wurde in einem sekurierten 250 ml Schlenk 9 g (0,01 mol) Diorganophosphitdichlorphosphit eingewogen, anschließend ausgeschleusst und in 75 mL getrocknetem Toluol gelöst. In einem zweiten sekurierten 250 ml Schlenk wurde 2,5 g (0,02 mol) 2,4-Dimethylphenol und 3 mL (0,022 mol) entgastes Triethylamin in 50 mL Toluol gelöst. Zur Phenol-Triethylamin Lösung wurde langsam und stetig bei Raumtemperatur innerhalb von 1,5 h das Chlorophosphit hinzugegeben. Die Reaktionsmixtur wurde über Nacht bei Raumtemperatur gerührt. Die Reaktionsmischung wurde auf 80 °C erwärmt. Nach 18 Stunden wurde die Reaktionsmischung gefrittet und das Filtrat wurde unter Ölpumpenvakuum bei 40 °C eingeengt. Anschließend wurde der Feststoff über 18 Stunden am Ölpumpenvakuum getrocknet. Dann wurde der Feststoff in 50 mL getrocknetem ACN gecrasht und gerührt. Der ausgefallene weiße Feststoff wurde dann abgefrittet. Reinheit 96%, Ausbeute 48%.

### Synthese (2) (Vergleichsligand)

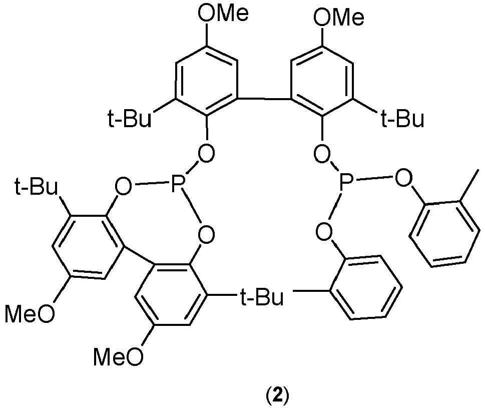

In der Glove-Box wurde in einem sekurierten 250 mL Schlenk 9 g (0,01 mol) Diorganophosphitdichlorphosphit eingewogen, anschließend ausgeschleusst und in 75 mL getrocknetem Toluol gelöst. In einem zweiten sekurierten 250 mL Schlenk wurden 2.2 g (2,1 mL 0,02 mol) 2-Methylphenol abgewogen und 12 h mittels Ölpumpenvakuum bei Raumtemperatur nachgetrocknet. Es wurde unter Rühren 50 mL getrocknetes Toluol und 3 mL = 2,2 g (0,022 mol) entgastes Triethylamin zugegeben und aufgelöst. Zur Phenol-Triethylamin Lösung wurde bei Raumtemperatur innerhalb 1,5 h das Dichlorophosphit hinzugegeben. Die Reaktionsmischung wurde 2 h bei Raumtemperatur gerührt und danach auf 80 °C erhitzt. Die Reaktionsmischung wurde 15 h bei dieser Temperatur gerührt und es wurden 3 mal 1,5 mL (0,011 mol) Triethylamin nachdosiert und 15 h weiter rühren gelassen. Das Ammoniumhydrochlorid wurde abgefrittet, 1 × mit 10 mL getrocknetem Toluol nachgewaschen bis zur Trockne eingeengt. Der Feststoff wurde 15 h bei Raumtemperatur getrocknet und mit 40 mL entgastem Acetonitril gerührt. Der ausgefallene weiße Feststoff wurde abgefrittet, der Schlenk wurde mit 2 mal 10 mL ACN nachgespült und nach dem Trocknen in die Glove-Box eingeschleust. Ausbeute 90%, Reinheit: 95%.

### Katalyseversuche

Die Hydroformylierung wurde in einem mit Druckkonstanthaltung, Gasflussmessung, und Begasungsrührer ausgestatteten 16 ml-Autoklaven der HEL group, Hertfordshire, Großbritannien, durchgeführt. Das als Substrat eingesetzte n-Octen (Oxeno GmbH, Octenisomerengemisch aus 1-Octen: 3 %; cis+trans-2-Octen: 49 %; cis+trans-3-Octen: 29 %; cis+trans-4-Octen: 16 %; gerüstisomere Octene: 3 %) wurde mehrere Stunden über Natrium am Rückfluss erhitzt und unter Argon destilliert.

Für die Versuche wurden die Reaktionslösungen vorab unter Argonatmosphäre vorbereitet. Hierfür wurden 0,0021 g Rh(acac)(CO)₂ und die entsprechende Menge an Phosphitverbindung eingewogen und mit 8,0 ml Toluol aufgefüllt. Die jeweils eingebrachte Masse an Toluol wurde für die GC-Analyse bestimmt. Anschließend wurden 1,80 g n-Octen (16 mmol) hinzugegeben. Die vorbereiteten Lösungen wurden anschließend in den Autoklaven eingefüllt und dieser dreimal mit Argon und dreimal mit Synthesegas (Linde; H₂ (99,999 %) : CO (99,997%) = 1:1) gespült. Dann wurde der Autoklav bei einem Gesamtdruck von 10 bar unter Rühren (900 U/min) auf die angestrebte Temperatur aufgeheizt. Nach Erreichen der Reaktionstemperatur wurde der Synthesegasdruck auf 20 bar erhöht und die Reaktion bei konstantem Druck für 4 h geführt. Der Autoklav wurde nach Ablauf der Reaktionszeit auf Raumtemperatur abgekühlt, unter Rühren entspannt und mit Argon gespült. Jeweils 0,5 ml der Reaktionsmischungen wurden nach Beenden der Reaktion entnommen, mit 4 ml Pentan verdünnt und gaschromatographisch analysiert: HP 5890 Series II plus, PONA, 50 m × 0,2 mm × 0,5 µm. Die quantitative Bestimmung von Restolefin und Aldehyd erfolgte gegen das Lösungsmittel Toluol als interner Standard.

### Ergebnisse der Katalyseversuche

### Reaktionsbedingungen:

[Rh]: 120 ppm, L:Rh = 1:2, p: 20 bar, T: 120 °C; t: 4 h

**Tabelle 1: Hydroformylierung der n-Octene**

| Ligand | n/iso-Selektivität in % |
|---|---|
| **1*** | 72 |
| **2** | 56 |

| | |
|---|---|
| * erfindungsgemäße Verbindung | |

### Definition der Selektivität:

Bei der Hydroformylierung gibt es die n/iso-Selektivität: das Verhältnis von linearem Aldehyd (= n) zu verzweigtem Aldehyd (= iso). Hierbei bedeutet die Selektivität bezüglich des n-Aldehyden, dass diese Menge an linearem Produkt gebildet wurde. Die restlichen Prozente entsprechen dann dem verzweigten Isomer. Bei einer Regioselektivität von 50% entstehen also n-Aldehyd und iso-Aldehyd zu gleichen Teilen.

Mit der erfindungsgemäßen Verbindung (1) konnte eine gegenüber dem Vergleichsliganden (2) gesteigerte n/iso-Selektivität erzielt werden.

Die durchgeführten Versuche belegen, dass die gestellte Aufgabe durch die erfindungsgemäße Verbindung gelöst wird.

## Patentansprüche

1. Verbindung gemäß der Struktur (**I**): 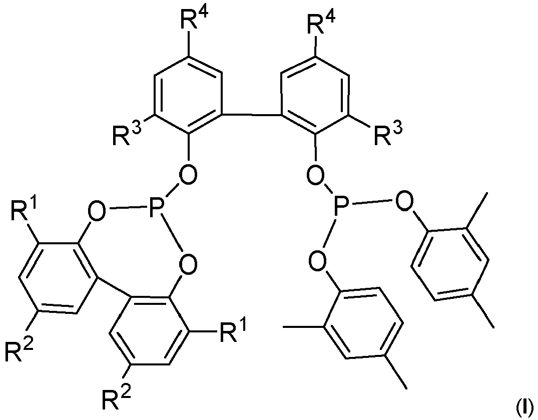 wobei R¹, R², R³, R⁴ ausgewählt sind aus: -H, -(C₁-C₁₂)-Alkyl, -O-(C₁-C₁₂)-Alkyl.

2. Verbindung nach Anspruch 1,
wobei R¹, R³ ausgewählt sind aus: -H, -(C₁-C₁₂)-Alkyl.

3. Verbindung nach einem der Ansprüche 1 oder 2,
wobei R¹, R³ für-(C₁-C₁₂)-Alkyl stehen.

4. Verbindung nach einem der Ansprüche 1 bis 3,
wobei R¹, R³ für den gleichen Rest stehen.

5. Verbindung nach einem der Ansprüche 1 bis 4,
wobei R², R⁴ ausgewählt sind aus: -H, -O-(C₁-C₁₂)-Alkyl.

6. Verbindung nach einem der Ansprüche 1 bis 5,
wobei R², R⁴ für -O-(C₁-C₁₂)-Alkyl stehen.

7. Verbindung nach einem der Ansprüche 1 bis 6,
wobei R², R⁴ für den gleichen Rest stehen.

8. Verbindung nach einem der Ansprüche 1 bis 7,
wobei R¹, R², R³, R⁴ nicht alle gleichzeitig für -CH₃ stehen.

9. Verbindung nach einem der Ansprüche 1 bis 8,
wobei die Verbindung die Struktur (1) aufweist:

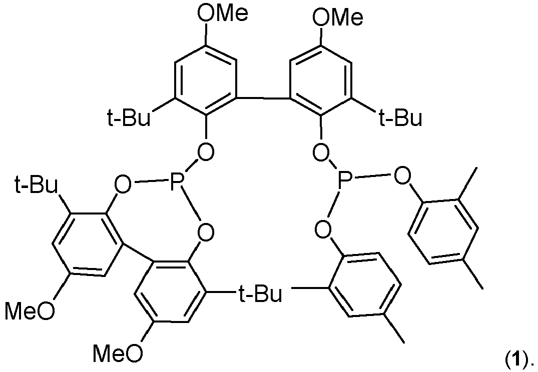

10. Verwendung einer Verbindung nach einem der Ansprüche 1 bis 9,
in einem Ligand-Metall-Komplex zur Katalyse einer Hydroformylierungsreaktion.

11. Verfahren umfassend die Verfahrensschritte:
a) Vorlegen eines Olefins,
b) Zugabe einer Verbindung nach einem der Ansprüche 1 bis 9 und einer Substanz, welche ein Metall ausgewählt aus: Rh, Ru, Co, Ir aufweist,
c) Zuführen von H₂ und CO,
d) Erwärmen des Reaktionsgemisches aus a) bis c), wobei das Olefin zu einem Aldehyd umgesetzt wird.

## Claims

1. Compound of the structure (I): 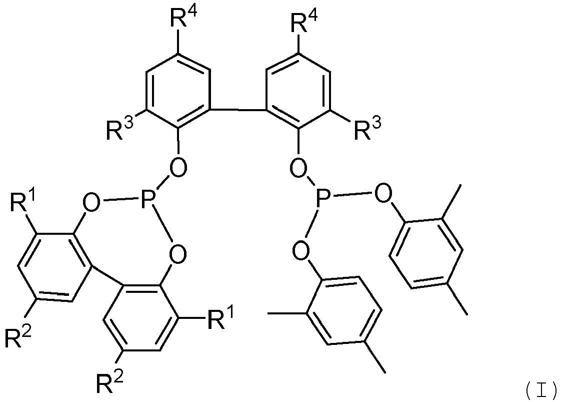 where R¹, R², R³, R⁴ are selected from: -H, -(C₁-C₁₂)-alkyl, -O-(C₁-C₁₂)-alkyl.

2. Compound according to Claim 1,
where R¹, R³ are selected from: -H, -(C₁-C₁₂)-alkyl.

3. Compound according to either of Claims 1 and 2,
where R¹, R³ are -(C₁-C₁₂)-alkyl.

4. Compound according to any of Claims 1 to 3,
where R¹, R³ are the same radical.

5. Compound according to any of Claims 1 to 4,
where R², R⁴ are selected from: -H, -O-(C₁-C₁₂)-alkyl.

6. Compound according to any of Claims 1 to 5,
where R², R⁴ are -O-(C₁-C₁₂)-alkyl.

7. Compound according to any of Claims 1 to 6,
where R², R⁴ are the same radical.

8. Compound according to any of Claims 1 to 7,
where R¹, R², R³, R⁴ are not all simultaneously -CH₃.

9. Compound according to any of Claims 1 to 8,
where the compound has the structure (1):

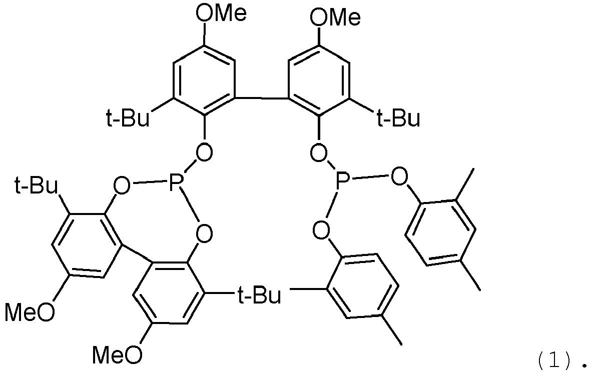

10. Use of a compound according to any of Claims 1 to 9 in a ligand-metal complex for catalysis of a hydroformylation reaction.

11. Process comprising the process steps of:
a) initially charging an olefin,
b) adding a compound according to any of Claims 1 to 9 and a substance containing a metal selected from: Rh, Ru, Co, Ir,
c) supplying H₂ and CO,
d) heating the reaction mixture from a) to c), with conversion of the olefin to an aldehyde.

## Revendications

1. Composé selon la structure (I) : 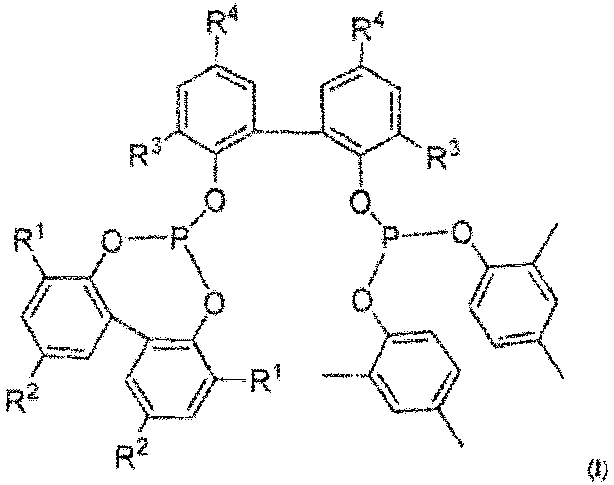 R¹, R², R³, R⁴ étant choisis parmi : -H, -(C₁₋₁₂)-alkyle, -O-(C₁₋₁₂)-alkyle.

2. Composé selon la revendication 1, R¹, R³ étant choisis parmi : -H, -(C₁₋₁₂)-alkyle.

3. Composé selon l'une quelconque des revendications 1 et 2, R¹, R³ représentant -(C₁₋₁₂)-alkyle.

4. Composé selon l'une quelconque des revendications 1 à 3, R¹, R³ représentant le même radical.

5. Composé selon l'une quelconque des revendications 1 à 4, R², R⁴ étant choisis parmi : -H, -O-(C₁₋₁₂)-alkyle.

6. Composé selon l'une quelconque des revendications 1 à 5, R², R⁴ représentant -O-(C₁₋₁₂)-alkyle.

7. Composé selon l'une quelconque des revendications 1 à 6, R², R⁴ représentant le même radical.

8. Composé selon l'une quelconque des revendications 1 à 7, R¹, R², R³, R⁴ ne représentant pas simultanément -CH₃.

9. Composé selon l'une quelconque des revendications 1 à 8, le composé présentant la structure (1) :

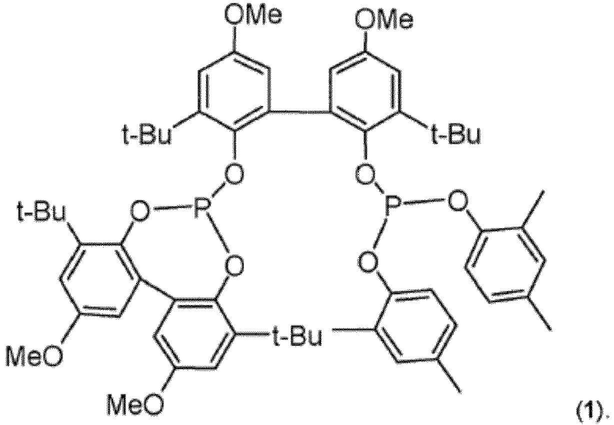

10. Utilisation d'un composé selon l'une quelconque des revendications 1 à 9, dans un complexe ligand-métal pour la catalyse d'une réaction d'hydroformylation.

11. Procédé comprenant les étapes de procédé :
a) chargement d'une oléfine,
b) ajout d'un composé selon l'une quelconque des revendications 1 à 9 et d'une substance qui présente un métal choisi parmi : Rh, Ru, Co, Ir,
c) introduction de H₂ et de CO,
d) chauffage du mélange réactionnel de a) à c), l'oléfine étant transformée en un aldéhyde.
